# EUROPÄISCHE PATENTSCHRIFT

(11) **EP 3 069 737 B1**
(45) Veröffentlichungstag und Bekanntmachung des Hinweises auf die Patenterteilung: **31.01.2018**
(21) Anmeldenummer: 16154344.2
(22) Anmeldetag: 04.02.2016
(51) Int. Cl.: A61M 1/00, B08B 15/00, A61B 18/04

(54) **VORRICHTUNG ZUR ABSAUGUNG VON CHIRURGISCHEM RAUCH**
DEVICE FOR EXTRACTING SURGICAL SMOKE
DISPOSITIF D'ASPIRATION DE FUMEES CHIRURGICALES

(30) Priorität: 04.02.2015 DE 102015101621
(43) Veröffentlichungstag der Anmeldung: 21.09.2016
(73) Patentinhaber: Innovations-Transfer Uphoff GmbH & Co. KG, 35039 Marburg an der Lahn (DE)
(72) Erfinder: Nink, Helmut, 65232 Taunusstein (DE)
(74) Vertreter: von Bülow & Tamada

(56) Entgegenhaltungen:
- WO-A2-02/38033
- US-A- 5 322 521
- US-A1- 2005 054 993

## Beschreibung

Die vorliegende Erfindung betrifft eine Vorrichtung zur Absaugung von chirurgischem Rauch und Arbeitskleidung für einen Operateur.
In der Chirurgie werden mit Hitze oder Ultraschall Gewebe zertrennt oder verschorft oder Blutungen gestillt. Hierzu kommen elektro- oder hochfrequenz-chirurgische (Hf-) Geräte, Laser oder Ultraschall-Skalpelle zum Einsatz. In der Regel entstehen dabei chirurgischer Rauch (Surgical Smoke, SS), sogenannte partikuläre und/oder gasförmige Emissionen, deren Partikelgrößen zwischen einigen Nanometern und 200 µm liegen. Ihre Gesundheitsschädlichkeit wird aufgrund von in vitro Versuchen und einzelnen Tierexperimenten angenommen.
Die partikulären Emissionen im chirurgischen Rauch können unter anderem biologische, zelluläre Anteile beinhalten und hängen insbesondere von der Intensität der Energieeinwirkung ab. Bei der Verdampfung von Gewebe mit Laser oder im Rahmen elektrochirurgischer Eingriffe sind im chirurgischen Rauch partikuläre Emissionen in Form von biologischen Substanzen belegt, die als intakte (Tumor-) Zellen, Zellfragmente, Blutzellen und virale Fragmente ebenso imponieren können, wie auch als lebensfähige Bakterien, was insbesondere auch für Staphylococcus aureus und Mycobacterium tuberculosis nachgewiesen wurde. Im Allgemeinen konnte für den mittleren Partikeldurchmesser der partikulären Emissionen im chirurgischen Rauch verfahrensspezifisch weniger als 0,1 µm bei Elektrokautern, zirka 0,3 µm bei Lasern und zirka 0,35 µm bis 6,5 µm bei Utraschallskalpellen ermittelt werden.

Neben den partikulären Emissionen sind im chirurgischen Rauch auch gas- bzw. dampfförmige Substanzen bekannt, die von exponierten Operateuren während der intraoperativen Arbeit als deutliche Geruchsbelästigungen wahrgenommen werden können. Ursächlich für diese Wahrnehmung sind anorganische (Kohlenstoffmonooxide und -dioxide, Schwefel- und Stickstoffoxide, Ammoniak etc.) und organische Stoffe (Benzol, Toluol, Ethylbenzol, Xylol, Cyanwasserstoff, Formaldehyd und polyzyklische aromatische Kohlenwasserstoffe etc.), wie diese bei jedem Verbrennungsprozess freigesetzt werden.

Für das medizinische Personal und insbesondere für den Operateur kann chirurgischer Rauch gesundheitliche Folgen haben, die sich beispielsweise in einer akuten Intoxikation, Kopfschmerzen, Schwächegefühl, Übelkeit, Muskelschwäche, Reizungen von Augen und Atemwegen zeigen können, auf die vor allem Asthmatiker empfindlich reagieren. Zudem können als gesundheitliche Folgen nach einer Vielzahl von Behandlungen, im Rahmen derer anogenitale Warzen oder Larynxpapillomatose von Papillomatosen abgetragen werden, Kehlkopfpapillomen auftreten.

Zur Minderung des Gesundheitsrisikos sind aus der Norm DIN 1946 Teil 4 raumlufttechnische Anlagen für Operationsräume bekannt. Hierbei handelt es sich um Vorrichtungen, die mit Außenluftvolumenströmen einen von den nationalen Vorgaben abhängigen Luftwechsel im Operationsraum herbeiführen und so den chirurgischen Rauch vom Operateur wegtransportieren. Für einen zehn- bis zwanzigfachen stündlichen Luftwechsel ist in einem durchschnittlichen Operationsraum zum Beispiel ein Außenluftvolumenstrom von mehr als 1.200 m³/h notwendig.

Weiterhin sind beispielsweise aus der US 5,431,650 A und der US 8,057,470 B2 mobile Vorrichtungen zur Absaugung von chirurgischem Rauch bekannt, welche den chirurgischen Rauch aufgesetzt auf ein Handstück eines elektrochirurgischen Gerätes an der Emissionsquelle lokal absaugen. Dabei bewertet die Berufsgenossenschaft die Wirksamkeit derartiger Vorrichtungen zur Absaugung von chirurgischem Rauch als zirka zwanzig bis vierzig mal weniger leistungsfähig, als die der raumlufttechnischen Anlagen.

Die Druckschrift US 5,192,276 offenbart eine Vorrichtung zur Absaugung von chirurgischem Rauch genannten partikulären und/oder gasförmigen Emissionen zwischen einer Emissionsquelle an der Brust eines Patienten und dessen Mund-Nase-Bereich, umfassend einen Absaugschlauch zur Bereitstellung eines Unterdrucks aus einer Unterdruckquelle, ein Absaugelement mit einer Kammer, einer die Kammer öffnenden Absaugöffnung, an die der Absaugschlauch zum Erzeugen des Unterdrucks in der Kammer anschließbar ist und einer die Kammer öffnenden Ansaugöffnung zum Ansaugen des chirurgischen Drucks basierend auf einem Unterdruck in der Kammer sowie ein Befestigungselement zur Befestigung des Absaugelementes am Patienten.

Die Druckschrift WO 2007/047664 A2 offenbart eine Vorrichtung zur Absaugung von chirurgischem Rauch genannten partikulären und/oder gasförmigen Emissionen zwischen einer Emissionsquelle im Frontbereich eines Operateurs und dessen Mund-Nase-Bereich, umfassend einen Absaugschlauch zur Bereitstellung eines Unterdrucks und einer Unterdruckquelle eine Absaugelement mit einer Kammer, einer die Kammer öffnenden Absaugöffnung, an die der Absaugschlauch zum Erzeugen des Unterdrucks in der Kammer anschließbar ist und einer die Kammer öffnenden Ansaugöffnung zum Ansaugen des chirurgischen Rauchs basierend auf dem Unterdruck.

Die Druckschrift US 4,865,049 offenbart eine Vorrichtung zur Absaugung von chirurgischem Rauch genannten partikulären und/oder gasförmigen Emissionen zwischen einer Emissionsquelle im Frontbereich eines Patienten und dessen Mund-Nase-Bereich umfassend einen Absaugschlauch zur Bereitstellung eines Unterdruckes aus einer Unterdruckquelle, ein Absaugelement mit einer Kammer, einer die Kammer öffnenden Absaugöffnung, an die der Absaugschlauch zum Erzeugen des Unterdrucks in der Kammer anschließbar ist und einer die Kammer öffnenden Ansaugöffnung zum Ansaugen des chirurgischen Rauchs basierend auf dem Unterdruck in der Kammer, ein Befestigungselement zur Befestigung des Absaugelements am Patienten und ein den chirurgischen Rauch stoppendes Abdeckelement, das winklig zu einer Befestigungsebene angeordnet ist, in der das Befestigungselement eingerichtet ist, das Absaugelement am Patienten zu befestigen. Eine Vorrichtung nach der Präambel von Anspruch 1 ist aus US2005054993 bekannt. Demgegenüber ist es Aufgabe der Erfindung, die bekannten Vorrichtungen zur Absaugung von chirurgischem Rauch zu verbessern. Die Aufgabe wird durch die Merkmale der unabhängigen Ansprüche gelöst. Bevorzugte Weiterbildungen sind Gegenstand der abhängigen Ansprüche. Gemäß einem Aspekt der Erfindung umfasst eine Vorrichtung zur Absaugung von chirurgischem Rauch genannten partikulären und/oder gasförmigen Emissionen zwischen einer Emissionsquelle im Frontbereich eines Operateurs und dessen Mund-Nasen-Bereich,
- einen Absaugschlauch zur Bereitstellung eines Unterdruckes aus einer Unterdruckquelle,
- ein Absaugelement mit
   - einer durch wenigstens einen schlauchförmigen Körper gebildeten Kammer mit einer Schlauchquerschnittsfläche,
   - einer die Kammer öffnenden Absaugöffnung, an die der Absaugschlauch zum Erzeugen des Unterdruckes in der Kammer anschließbar ist, und
   - einer Vielzahl die Kammer öffnenden und den Mantel des schlauchförmigen Körpers durchdringenden Ansaugöffnungen zum Ansaugen des chirurgischen Rauchs basierend auf dem Unterdruck in der Kammer,
- wobei jede Ansaugöffnung eine Öffnungsquerschnittsfläche aufweist und wobei eine Summe aller Öffnungsquerschnittsflächen kleiner ist als die 2,5-fache Schlauchquerschnittsfläche.

Der angegebenen Vorrichtung liegt die Überlegung zugrunde, dass zwar einige der eingangs genannten Vorrichtungen einen schlauchförmigen Körper besitzen, an diesem aber die Absaugwirkung ungleich verteilt ist. Das liegt daran, dass der Ansaugdruck an den Ansaugöffnungen, die näher am Absaugschlauch liegen, höher ist als an Ansaugöffnungen, die vom Absaugschlauch weiter entfernt sind. Aufgrund dieser ungleichen Druckverteilung ist eine effektive Absaugwirkung durch die Vorrichtung nicht in jeder Position des Operateurs sichergestellt. Entgegen den Einsatzszenarien der eingangs genannten Vorrichtungen soll die angegebene Vorrichtung jedoch an einem sich bewegenden Operateur verwendet werden, ohne dass diese allzu groß dimensioniert werden muss, um den Operateur bei der Arbeit nicht zu stören.

Hier greift die angegebene Vorrichtung mit dem Vorschlag an, die einzelnen Ansaugöffnungen analog zu kritischen Düsen auszuführen. Dies wird dadurch erreicht, dass die Summe aller Öffnungsquerschnittsflächen der Ansaugöffnungen kleiner ist, als die 2,5-fache Schlauchquerschnittsfläche. Auf diese Weise können zum Betreiben der angegebenen Vorrichtung die in der DIN 1946 Teil 4 für raumlufttechnische Anlagen definierten Geräte zum Erzeugen des Unterdrucks in der angegebenen Vorrichtung verwendet und jede Ansaugöffnung als kritische Düse ausgebildet werden. Durch die Ausbildung der Ansaugöffnungen im Sinne von kritischen Düsen wird sichergestellt, dass die Druckverteilung über den gesamten schlauchförmigen Körper gleich ist, und mit steigendem Abstand vom Ansaugschlauch nicht abnimmt.

Um die Ansaugwirkung selbst nicht all zu stark zu verkleinern, sollte die Summe der Querschnittsflächen der einzelnen Ansaugöffnungen nicht all zu klein gewählt werden, weshalb die Summe aller Öffnungsquerschnittsflächen zweckmäßigerweise aus einem Bereich zwischen dem 0,5-fachen und dem 2,5-fachen der Schlauchquerschnittsfläche ausgewählt werden sollte.

Ferner kann die angegebene Vorrichtung ein Befestigungselement zur Befestigung des Absaugelementes am Operateur umfassen.

Der angegebenen mobilen Vorrichtung liegt die Überlegung zugrunde, dass die Randbedingungen für die eingangs genannten Vorrichtungen zur Absaugung von chirurgischem Rauch intraoperativ sehr variabel sind. So muss beispielsweise der Abstand der Ansaugöffnung zur Emissionsquelle stets neu angepasst werden, um eine zufriedenstellende Wirksamkeit einer eingangs genannten Vorrichtung zur Absaugung von chirurgischem Rauch zu gewährleisten.

Zwar könnte die eingangs genannte Vorrichtung zur Absaugung von chirurgischem Rauch auch als stationäre Wandabsaugeinrichtung ausgebildet werden. Der Abstand der Ansaugöffnung zur Emissionsquelle des chirurgischen Rauchs wäre in diesem Fall aber so hoch, dass zur Erfüllung einer ausreichend hohen Absaugwirkung sehr hoher Volumenstrom notwendig wäre. Dieser Volumenstrom muss in Abhängigkeit des Eingriffes dimensioniert werden, in den die Art der medizinischen Behandlung und der Umfang des Eingriffs sowie die betroffene Körperstelle und das betroffene Gewebe eingehen. Vereinfacht gesagt muss bei der Dimensionierung einer Vorrichtung zur Absaugung von chirurgischem Rauch von der größten anzunehmenden Gesamtexposition des Operationsteams ausgegangen werden, um diese unterhalb eines vorbestimmten Grenzwertes zu halten.

Auch schränkt ein hoher Volumenstrom die Handhabbarkeit einer eingangs genannten Vorrichtung zur Absaugung chirurgischen Rauchs ein, so dass diese nicht beliebig zu dimensionieren ist.

Hier greift die angegebene Vorrichtung zur Absaugung von chirurgischem Rauch mit dem Vorschlag an, diese am Operateur zu befestigen und weist ein Befestigungselement zur Befestigung am Operateur auf. Auf diese Weise kann ein konstanter und geringer Abstand der Ansaugöffnung zur Emissionsquelle erreicht werden, so dass mit einem vergleichsweise geringen Volumenstrom eine hohe Absaugwirkung erzielt werden kann. Von diesem konstanten und geringen Abstand kann gerade beim Operateur ausgegangen werden, weil dieser seine Position während der Operation in der Regel nicht oder nur wenig ändert.

In einer Weiterbildung der angegebenen Vorrichtung zur Absaugung von chirurgischem Rauch ist die Kammer durch wenigstens einen schlauchförmigen Körper gebildet. Durch die schlauchförmige Ausführung der Kammer kann diese beispielsweise in einer Bauchgegend des Operateurs an diesen vergleichbar mit einem Gürtel angelegt und nah an die Emissionsquelle des chirurgischen Rauches herangeführt werden. Der schlauchförmige Körper bietet einerseits ausreichend Platz für eine Befestigung der Kammer am Operateur, andererseits kann die Kammer aber auch ausreichend klein ausgebildet werden, so dass die Unterdruckquelle nicht übermäßig groß ausgebildet werden muss, um einen ausreichend hohen Unterdruck in der Kammer zu erzeugen.

Die Ansaugöffnungen können am schlauchförmigen Körper grundsätzlich beliebig, d.h. ein- oder mehrseitig, angeordnet werden. In einer besonderen Weiterbildung der angegebenen Vorrichtung zur Absaugung von chirurgischem Rauch durchdringen die Ansaugöffnungen den Mantel des schlauchförmigen Körpers. Anders als in den eingangs genannten Vorrichtungen zur Absaugung chirurgischen Rauchs steht auf dem Mantel eine große Fläche zur Verfügung, über die mehrere Ansaugöffnungen verteilt werden können, um den chirurgischen Rauch von der Emissionsquelle über einen großen Wirkungsbereich anzusaugen.

Die Kammer kann dabei aus mehreren schlauchförmigen Körpern zusammengesetzt sein. In einer bevorzugten Weiterbildung der angegebenen Vorrichtung zur Absaugung chirurgischen Rauchs ist die Kammer durch wenigstens einen weiteren schlauchförmigen Körper gebildet, der vorzugsweise winklig zum ersten schlauchförmigen Körper angeordnet ist. Einer der beiden schlauchförmigen Körper kann dann beispielsweise horizontal zu einer Bodenfläche des Operationsraumes angeordnet sein, während der andere schlauchförmige Körper zur Bodenfläche vertikal angeordnet sein kann. Der horizontal angeordnete schlauchförmige Körper stellt dann eine Art Grobabsauger (auch Grobeliminator oder Grobfilter genannt) für den chirurgischen Rauch aus der Emissionsquelle dar, der diesen in einer Horizontalebene allgemein für alle Anwesenden im Operationsraum entfernt. Demgegenüber wirkt der vertikal angeordnete Schlauchkörper dann als Feinabsauger (auch Feineliminator oder Feinfilter genannt), der Reste des durch die Horizontalebene durchtunnelnden chirurgischen Rauchs auf dem Weg zur Nase des Operateurs entfernt. Alle anderen Anwesenden im Operationsraum benötigen diesen Feinfilter aufgrund ihrer Distanz zur Emissionsquelle in der Regel nicht.

Der schlauchförmige Körper kann im Profil jede beliebige Form mit Ecken und/oder Rundungen aufweisen. So ist als Profilform beispielsweise eine Kreisform, eine Ellipsenform, eine Dreiecksform, eine beliebige Vierecksform, eine Eistütenform oder dergleichen möglich. Insbesondere Profile mit Rundungen, wie die Kreis- oder Ellipsenform, sind hierbei besonders günstig, weil die Ansaugöffnung am Mantel des schlauchförmigen Körpers exakt in einer Richtung ausrichtbar ist, in der der chirurgische Rauch mit einer hohen Wirkung angesaugt werden kann.

Die ausgerichtete Ansaugöffnung kann in einer besonders bevorzugten Weiterbildung der angegebenen Vorrichtung zur Absaugung von chirurgischem Rauch zu einer Befestigungsebene, in der das Befestigungselement eingerichtet ist, das Absaugelement am Operateur zu befestigen, mit einem Winkel zwischen -90° und 90°, insbesondere 45°, verlaufen.

In einer zusätzlichen Weiterbildung der angegebenen Vorrichtung zur Absaugung von chirurgischem Rauch besitzt das Absaugelement wenigstens eine weitere, die Kammer öffnende Ansaugöffnung zum Ansaugen des chirurgischen Rauchs basierend auf dem Unterdruck in der Kammer. Letztendlich kann die Anzahl der Ansaugöffnungen zum Ansaugen des chirurgischen Rauches in die Kammer beliebig sein, solange sichergestellt ist, dass die Unterdruckquelle in der Kammer einen wirksamen Unterdruck aufbauen kann. Hierzu hat sich gezeigt, dass die einzelnen Ansaugöffnungen untereinander einen Mindestabstand von 4 mm und jeweils eine maximale Querschnittsfläche von 3 mm² bis 4 mm² aufweisen sollten.

In einer anderen Weiterbildung der angegebenen Vorrichtung zur Absaugung von chirurgischem Rauch ist das Befestigungselement eingerichtet, das Absaugelement selbstklebend am Operateur zu befestigen. Auf diese Weise kann der Operateur die Vorrichtung zu Beginn der Operation in einfacher Weise an sich befestigen und nach der Operation in ebenso einfacher Weise wieder entfernen. Hierzu umfasst das Befestigungselement vorzugsweise einen Folienkörper.

In einer noch anderen Weiterbildung umfasst die angegebene Vorrichtung zur Absaugung von chirurgischem Rauch ein Abdeckelement, das winklig, insbesondere rechtwinklig, zu einer Befestigungsebene angeordnet ist, in der das Befestigungselement eingerichtet ist, das Absaugelement am Operateur zu befestigen. Das Abdeckelement stellt für den aus der Emissionsquelle ausgestoßenen chirurgischen Rauch ein Hindernis dar, erzeugt so eine turbulente Strömung und verlangsamt damit die Geschwindigkeit des chirurgischen Rauchs, mit der der chirurgische Rauch das Absaugelement passiert. Auf diese Weise kann der chirurgische Rauch besser vom Ansaugelement erfasst werden, was die Wirksamkeit der angegebenen Vorrichtung weiter erhöht.

In einer zusätzlichen Weiterbildung der angegebenen Vorrichtung umfasst das Abdeckelement mehrere zueinander verschiebliche Teile, d.h. dass das Abdeckelement mehrgliedrig aufgebaut sein kann, wodurch es individuell an die Körperform des Operateurs angepasst werden kann. Die Form der einzelnen verschieblichen Teile kann beliebig ausgebildet werden. Als besonders günstig hat sich dabei eine Trapezform herausgestellt. Alternativ wäre auch eine Rautenform möglich.

Das Material des Absaugelementes kann grundsätzlich beliebig ausgeführt werden. Wird das Absaugelement beispielsweise aus hygienischen Gründen als Einwegartikel ausgeführt, so bietet sich ein leicht und günstig zu verarbeitender Kunststoff an. Soll das Absaugelement mehrmals verwendet werden, so bietet sich ein Edelstahl an, der in einfacher Weise steril gehalten werden kann.

Ein weiterer Aspekt der Erfindung umfasst Arbeitskleidung für einen Operateur, ein Kleidungsstück zur wenigstens teilweisen Bedeckung des Operateurs und eine der angegebenen Vorrichtungen, die über ihr Befestigungselement an einer vom Operateur aus gesehenen Außenseite des Kleidungsstückes an diesem befestigt ist. Das Kleidungsstück kann grundsätzlich beliebig, beispielsweise als Schutzschürze oder als Gürtel ausgeführt sein. Dabei kann die angegebene Vorrichtung fest oder lösbar an dem Kleidungsstück befestigt sein.

In einer Weiterbildung umfasst die angegebene Arbeitskleidung ein zwischen dem Befestigungselement und dem Kleidungsstück angeordnetes Trägerelement. Dieses Trägerelement kann selbstklebend und/oder seinerseits als Zuggurt ausgeführt.

Das Trägerelement kann aus einem Material mit einer glatten Oberfläche, wie beispielsweise einem Kunststoff, insbesondere Polypropylen oder Papier, gefertigt sein. Versuche haben gezeigt, dass die Eliminationswirkung des chirurgischen Rauchs durch Verwendung eines derartigen glatten Trägerelementes um mehr als 40% gesteigert werden konnte. Das Trägerelement sollte sich dabei von einem Schulterbereich des Operateurs bis unterhalb der Emissionsquelle erstrecken.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit der Zeichnung näher erläutert werden. Es zeigen:
- Fig. 1 und 2: einen schematischen Versuchsaufbau zur Erzeugung chirurgischen Rauchs,
- Fig. 3 und 4: eine schematische Darstellung des Versuchsaufbaus aus den Fig. 1 und 2 im Einsatz,
- Fig. 5: eine schematische Darstellung des Betriebs einer raumlufttechnischen Anlage nach DIN 1946 Teil 4 in dem Versuchsaufbau der Fig. 1 und 2,
- Fig. 6: die Absaugwirkung der raumlufttechnischen Anlage aus Fig. 5 über ihren Volumenstrom,
- Fig. 7 und 8: ein Ausführungsbeispiel einer Vorrichtung gemäß der vorliegenden Erfindung,
- Fig. 9: die Absaugwirkung der Vorrichtung aus Fig. 7 und 8 über ihren Abstand zur Emissionsquelle,
- Fig. 10: die Absaugwirkung der Vorrichtung aus Fig. 7 und 8 über ihren Volumenstrom,
- Fig. 11 bis 14: verschiedene alternative Ausführungsformen der Vorrichtung aus Fig. 7,
- Fig. 15: einen Teil der angegebenen Vorrichtung aus Fig. 7 im Schnitt,
- Fig. 16: eine Weiterbildung der angegebenen Vorrichtung aus Fig. 7,
- Fig. 17: die angegebene Vorrichtung auf einem Trägerelement,
- Fig. 18A bis 20K: ein Absaugelement der angegebenen Vorrichtung in verschiedenen Ausführungen,
- Fig. 21A bis 22C: ein Abdeckelement der angegebenen Vorrichtung in verschiedenen Ausführungen, und
- Fig. 23: ein weiteres Ausführungsbeispiel der angegebenen Vorrichtung.

In den Figuren werden gleiche technische Elemente mit gleichen Bezugszeichen versehen und nur einmal beschrieben. Die Figuren sind rein schematisch und geben vor allem nicht die tatsächlichen geometrischen Verhältnisse wieder.

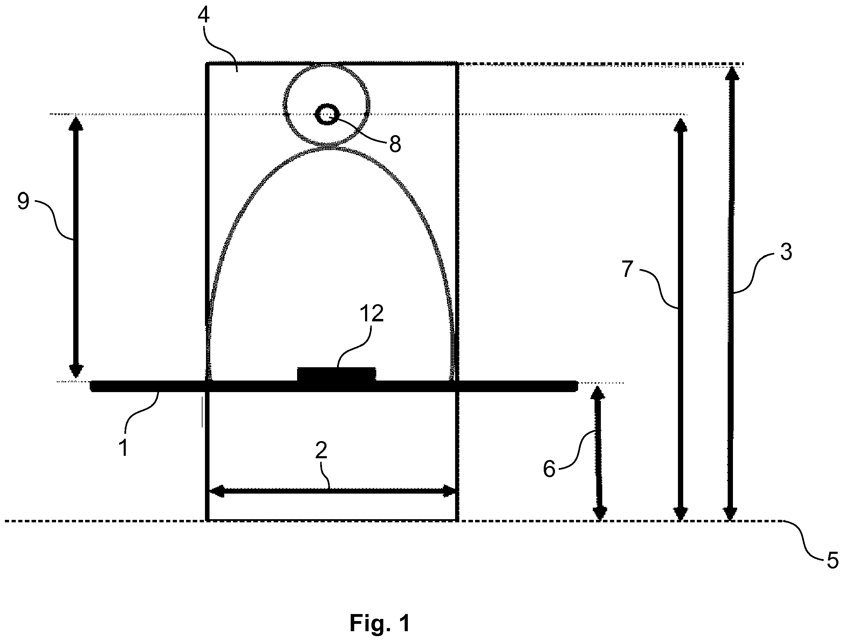

Zunächst soll anhand der Fig. 1 bis 2 ein Versuchsaufbau beschrieben werden. In diesem Versuchsaufbau kann die angegebene Vorrichtung getestet werden, worauf an späterer Stelle näher eingegangen wird.

Der Versuchsaufbau umfasst einen Operationstisch 1, an dem seitlich ein Brett mit einer Brettbreite 2 von 0,5 m und einer Bretthöhe 3 von 2 m angeordnet ist. Dieses Brett soll einen Operateur 4 simulieren, dessen Konturen der Übersichtlichkeit halber auf dem Brett aufgetragen sind. In einigen der nachstehenden Figuren sind teilweise nur die Konturen des Operateurs 4 aufgezeichnet.

Der Operateur 4 steht auf einem gestrichelt dargestellten Boden 5. Der Operationstisch 1 ist über dem Boden 5 mit einer Tischhöhe 6 von 1,2 m angeordnet. Der Operateur 2 soll auf einer Inhalationshöhe 7 von 1,6 m eine Inhalationsöffnung 8 besitzen, die einen Mund-/Nasenbereich des Operateurs 2 simuliert. Der Inhalationsabstand 9 des Operateurs 4 vom Operationstisch 1 beträgt damit 0,4 m.

Die Inhalation des Operateurs 4 wird mit einem Laser-Partikel-Monitor 10 gemessen, der mit der Inhalationsöffnung 8 über einen Messschlauch 11 verbunden ist.

Auf dem Operationstisch 1 befindet sich ein zu operierendes Testobjekt 12 in Form eines Gewebestückes. Als Testobjekt 12 kann beispielsweise frisches Muskelgewebe eines Schweines verwendet werden, was für das vorliegende Ausführungsbeispiel angenommen werden soll.

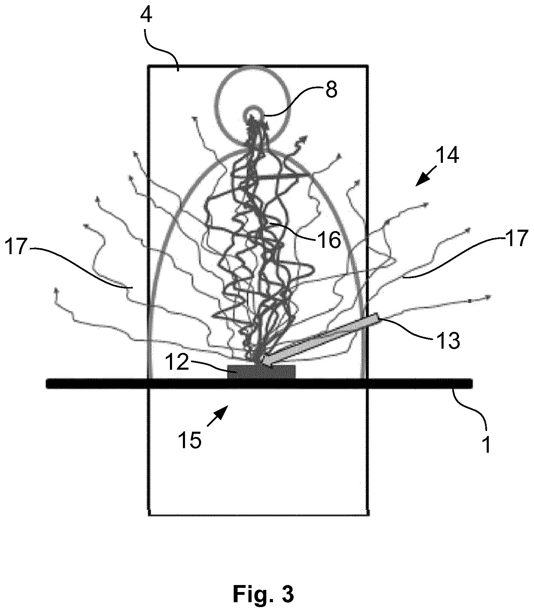

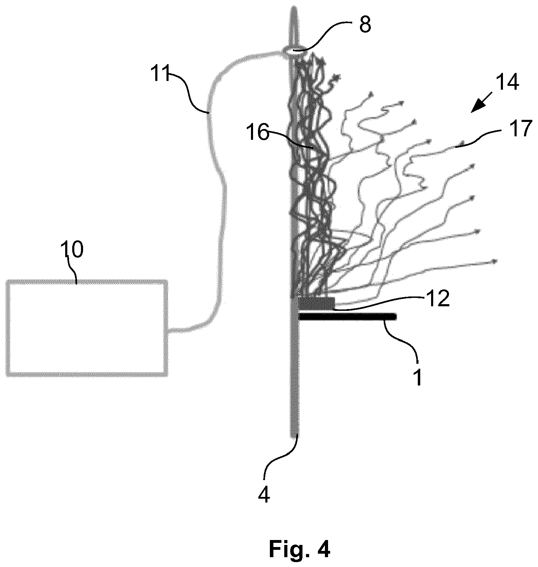

Das Testobjekt 12 kann in einer anhand der Fig. 3 und 4 beschriebenen Weise mit einem in Fig. 3 durch einen Pfeil angedeuteten Arbeitsgerät 13 bearbeitet werden. Als Arbeitsgerät 13 kann ein bipolares Hochfrequenz-Chirurgie-Gerät mit einer Leistung von 70 Watt zusammen mit einer Kugelelektrode verwendet werden. Um reproduzierbare Versuchsergebnisse zu erhalten, sollte die Bearbeitung des Testobjekts 12 standardisiert durchgeführt werden. Hierzu kann beispielsweise mit dem zuvor erwähnten Arbeitsgerät 13 ein Schnitt in das Testobjekt 12 mit einer Schnittlänge von 5 cm und einer Schnittzeit von 5 sec eingeschnitten werden. Wird das Testobjekt 12 in der zuvor erläuterten Weise bearbeitet, werden als chirurgischer Rauch 14 50.000.000 Partikel mit einer Größe von gleich oder mehr als 0,5 µm freigesetzt. Das Testobjekt 12 und das Arbeitsgerät 13 simulieren somit eine definierte Emissionsquelle 15 für den chirurgischen Rauch 14.

Der chirurgische Rauch 14 verbreitet sich fächerförmig im Raum, wobei in Fig. 3 deutlich zu sehen ist, wie sich der chirurgische Rauch 14 durch die Inhalation des Operateurs 4 im Bereich seiner Inhalationsöffnung 8 sammelt. Nachstehend soll der sich ausbreitende chirurgische Rauch 14 in drei Bereiche unterschieden werden. Während der sich zur Inhalationsöffnung 8 ausbreitende chirurgische Rauch 14 als heißer chirurgischer Rauch 16 bezeichnet werden soll, soll der (kältere) sich seitlich ausbreitende chirurgische Rauch 14 als seitlicher chirurgischer Rauch 17 bezeichnet werden. Die Fig. 3 und 4 verdeutlichen dabei, dass sich der seitliche chirurgische Rauch 14 nach allen Seiten ausbreitet.

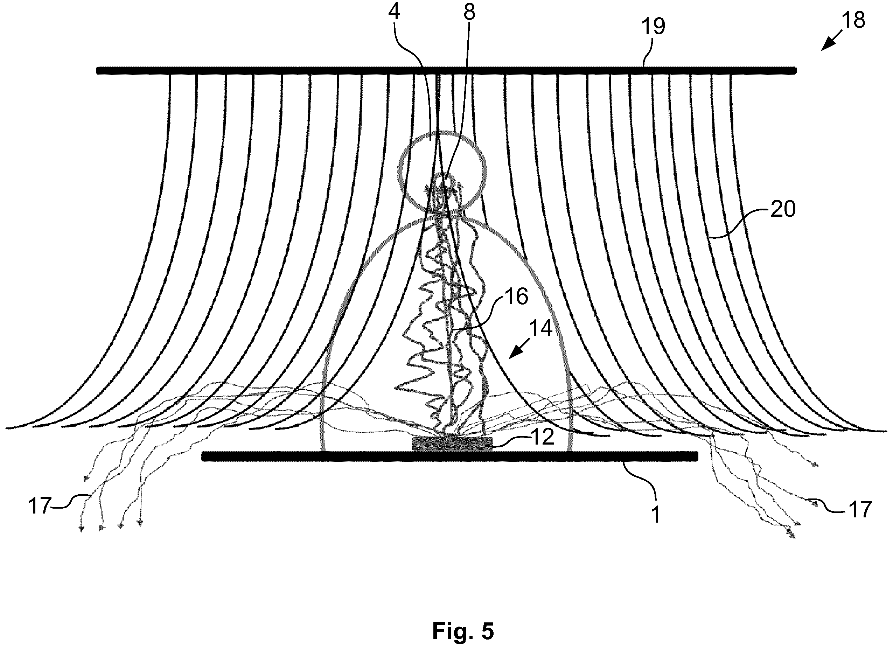

Zur Reduktion des chirurgischen Rauchs 14 kann nach DIN 1946 Teil 4 eine in Fig. 5 angedeutete raumlufttechnische Anlage 18 zum Einsatz kommen. Eine derartige raumlufttechnische Anlage 18 umfasst eine Zuluftdecke 19, die gegen den nicht weiter dargestellten Boden 5 einen Deckenluftstrom 20 ausbläst, der den chirurgischen Rauch 14 von der Inhalationsöffnung 8 weg gegen den Boden 5 drückt.

Die Zuluftdecke 19 stellt jedoch grundsätzlich eine Druckquelle dar, während die Inhalationsöffnung eine Drucksenke ist, was dazu führt, dass der Deckenluftstrom 20 im Bereich der Inhalationsöffnung 8 zumindest teilweise neutralisiert wird. Weiterhin erfährt der heiße chirurgische Rauch 16 gegenüber der kälteren Raumluft einen erheblichen Auftrieb. Wie in Fig. 5 deutlich zu sehen, führt dies dazu, dass nur der (kältere) seitliche chirurgische Rauch 17 vollständig gegen den Boden 5 gedrückt wird, während der heiße chirurgische Rauch 16 durch den Deckenluftstrom 20 weniger stark beeinflusst wird und sich so weiterhin zumindest teilweise zur Inhalationsöffnung 8 ausbreiten kann. Dieses Phänomen wird durch die Geometrie des Operateurs 4 und seine (zum Operationstisch 1 gebeugte) Arbeitshaltung noch weiter verstärkt, die im vorliegenden Ausführungsbeispiel aufgrund der brettförmigen Ausgestaltung des Operateurs nicht berücksichtigt sind.

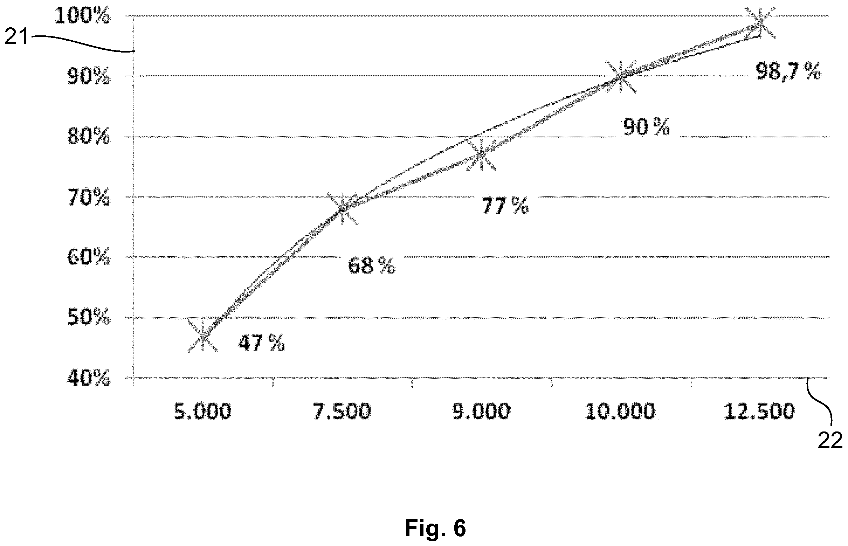

Um die Ausbreitung des heißen chirurgischen Rauchs 16 zur Inhalationsöffnung 8 möglichst vollständig zu vermeiden, sollte der Deckenluftstrom 20 möglichst leistungsstark ausgebildet sein. Dies ist in Fig. 6 verdeutlicht, in dem die Absaugwirkung 21 in Prozent über den von der Leistung des Deckenluftstromes 20 abhängen Volumenluftstrom 22 des Deckenluftstromes 20 in m³/h aufgetragen ist. Die Absaugwirkung 21 beschreibt, wie viele der anfangs genannten aus der Emissionsquelle 15 ausgestoßenen 50.000.000 Partikel des chirurgischen Rauches 14 die Inhalationsöffnung 8 erreichen.

Wie aus Fig. 6 zu sehen, erreichen selbst bei einem sehr starken Volumenstrom 22 von 12.000 m³/h immer noch 98 % der aus der Emissionsquelle 15 ausgestoßenen Partikel des chirurgischen Rauchs 14 den Inhalationsbereich 8 des Operateurs 4. Je länger eine Operation dauert und je öfter das Testobjekt 12 in der zuvor erläuterten Weise bearbeitet wird, desto höher ist letztendlich die Gesamtbelastung für den Operateur 4 und gegebenenfalls andere Anwesende im Operationsraum. Andererseits kann aber aus Gründen der Behaglichkeit für das Operationsteam der Volumenstrom 22 nicht beliebig hoch eingestellt werden, um diese Gesamtbelastung stets unterhalb eines vorbestimmten Grenzwertes zu halten.

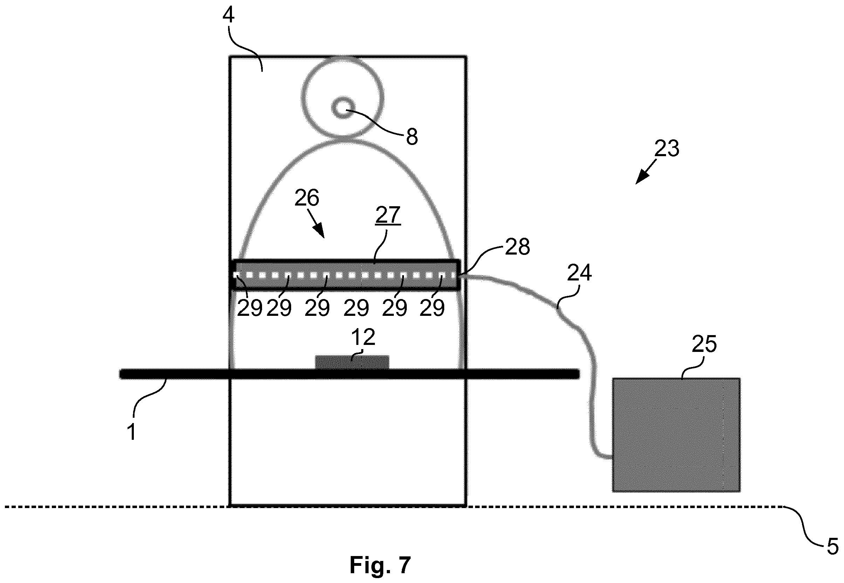

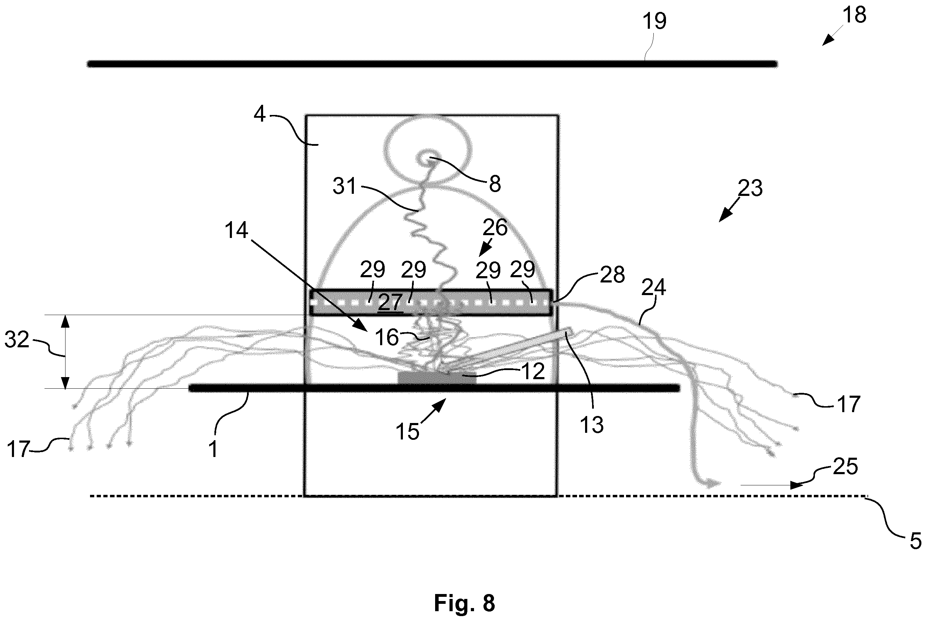

Hier greift das vorliegende Ausführungsbeispiel mit dem Vorschlag an, zwischen die Inhalationsöffnung 8 und die Emissionsquelle 15 mit dem Testobjekt 12 eine weitere Drucksenke einzuführen, die den heißen chirurgischen Rauch 16 aufnimmt, bevor dieser die als Drucksenke wirkende Inhalationsöffnung 8 erreicht. Dieser Vorschlag soll nachstehend anhand der Fig. 7 und 8 zunächst grundsätzlich erläutert werden.

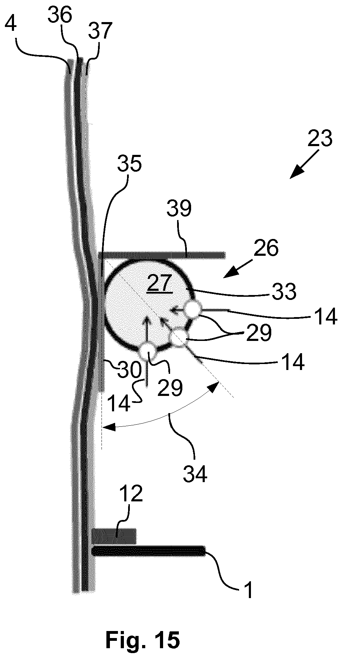

Die weitere Drucksenke ist eine Vorrichtung 23 zur Absaugung des chirurgischen Rauchs 14. Sie umfasst einen Absaugschlauch 24 zur Bereitstellung eines Unterdruckes aus einer Unterdruckquelle 25. Ferner umfasst die Vorrichtung 23 ein Absaugelement 26 mit einer Kammer 27, einer die Kammer 27 öffnenden Absaugöffnung 28, an die der Absaugschlauch 24 zum Erzeugen des Unterdruckes in der Kammer 27 anschließbar ist, und mehreren die Kammer 27 öffnenden Ansaugöffnungen 29 zum Ansaugen wenigstens des heißen chirurgischen Rauchs 16 basierend auf dem Unterdruck in der Kammer 27. Von diesen Ansaugöffnungen 29 sind in den Fig. 7 und 8 der Übersichtlichkeit halber nicht alle mit einem Bezugszeichen versehen. Schließlich umfasst die Vorrichtung 23 ein erst später in Fig. 15 diskutiertes Befestigungselement 30 zur Befestigung des Absaugelementes 26 am Operateur 4.

Durch die vorgeschlagene Vorrichtung 23 werden insbesondere die heißen chirurgischen Rauche 16 über die Ansaugöffnungen 29 abgesaugt, bevor diese den Inhalationsbereich 8 des Operateurs 4 erreichen können. Ein gegebenenfalls die Vorrichtung 23 passierender Rest 31 an chirurgischem Rauch 14, der die vorgeschlagene Vorrichtung 23 dennoch passiert, kann in Abhängigkeit der Operationsdauer und der Anzahl der durchgeführten, zur Emission des chirurgischen Rauchs 14 führenden Operationstätigkeiten gegebenenfalls akzeptiert werden.

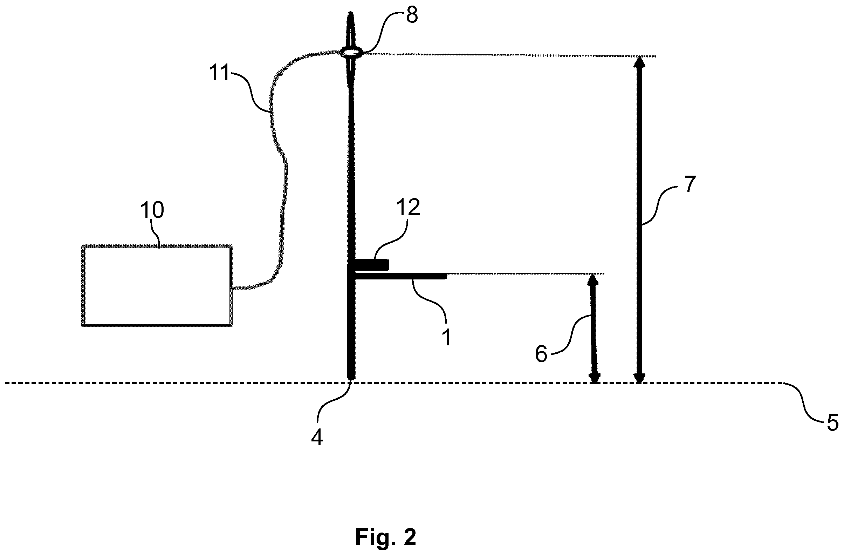

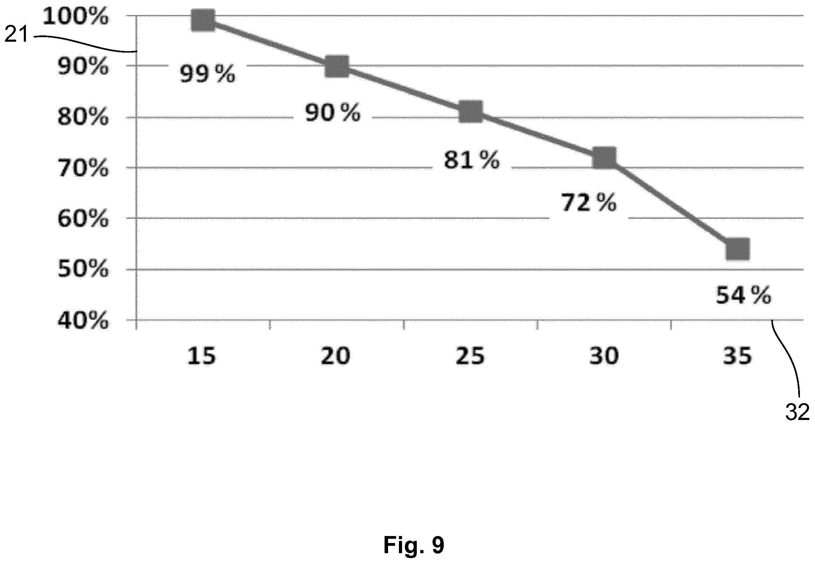

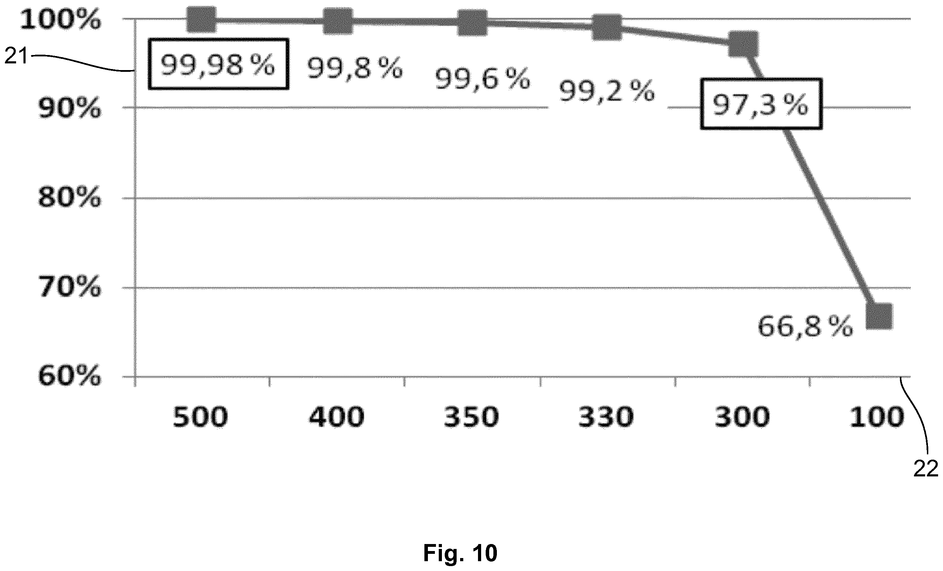

Die Wirkung der angegebenen Vorrichtung 23 konnte in dem Versuchsaufbau der Fig. 1 und 2 experimentiell nachgewiesen werden. Ein entsprechendes Experiment wird nachstehend anhand der Fig. 9 und 10 näher erläutert.

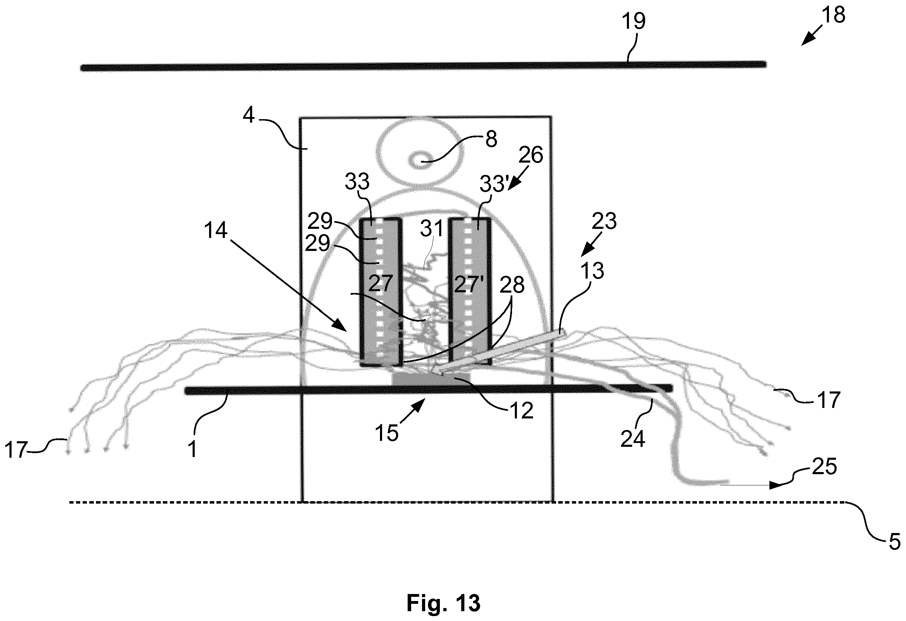

Für das Experiment ist Kammer 27 aus einem schlauchförmigen Körper 33 mit einem Schlauchdurchmesser von 22 mm gebildet. Die einzelnen Ansaugöffnungen 29 sind auf dem Schlauchmantel über eine Länge von 400 mm auf einer Linie angeordnet und durchdringen ihn in einem nicht weiter dargestellten Abstand von 4 mm. Auf diese Weise besitzt die angegebene Vorrichtung 23 insgesamt 95 Ansaugöffnungen 29. Die einzelnen Ansaugöffnungen 29 sind für das Experiment kreisförmig mit einem Durchmesser von 2 mm ausgeführt. Zudem sind die Ansaugöffnungen 29 mit einem in Fig. 13 angedeuteten Ansaugwinkel 34 von 45° vom Operateur 4 weg auf die Emissionsquelle 15 gerichtet. Die angegebene Vorrichtung 23 selbst ist in einer zunächst variablen Ansaughöhe 32 über der Emissionsquelle 15 angeordnet. Für das Experiment wird aus der Zuluftdecke 19 in Fig. 8 nicht gezeigter Weise ein Deckenluftstrom 20 von 7.500 m³/h ausgegeben.

Zunächst soll anhand Fig. 9 die Absaugwirkung 21 der angegebenen Vorrichtung 23 mit einem konstanten, durch den Absaugschlauch 24 strömenden Volumenstrom 22 von 330 l/min (entspricht 19,3 m³/h) betrachtet werden. Hierzu ist in Fig. 9 die Absaugwirkung 21 über die zunächst variable Ansaughöhe 32 aufgetragen, die in Fig. 9 in cm angegeben ist.

Deutlich zu sehen ist, wie die Absaugwirkung 21 zunimmt, je näher die angegebene Vorrichtung 23 der Emissionsquelle 15 kommt. Ihre maximale Absaugwirkung 21 erreicht die angegebene Vorrichtung 23 in einer Ansaughöhe 32 von 15 cm (entspricht 0,15 m) über der Emissionsquelle 15.

In dieser maximalen Ansaughöhe 32 von 15 cm soll nun der Volumenstrom 22 durch den Absaugschlauch 24, der in Fig. 10 in l/min angegeben ist, gemäß Fig. 10 variiert werden. Hier zeigt sich, dass bei einem Volumenstrom 22 von 500 l/min (entspricht 30 m³/h) eine Absaugwirkung 21 erreicht werden kann, bei der von den aus der Emissionsquelle 15 emittierten 50.000.000 Partikeln des chirurgischen Rauchs 14 bis zu 99,98% vor Erreichen der Inhalationsöffnung 8 abgesaugt werden können. Bei geeigneter Auslegung der angegebenen Vorrichtung können die die Inhalationsöffnung 8 erreichenden Partikel des chirurgischen Rauchs 14 bis auf wenige 100 Partikel gesenkt werden.

Insbesondere zeigt sich aber, dass die angegebene Vorrichtung 23 mit einem deutlich geringeren Volumenstrom 22 eine deutlich höhere Absaugwirkung 21 (auch Filter- oder Eliminationswirkung oder -leistung genannt) erreichen kann als die raumlufttechnische Anlage 18 mit der Zuluftdecke 19.

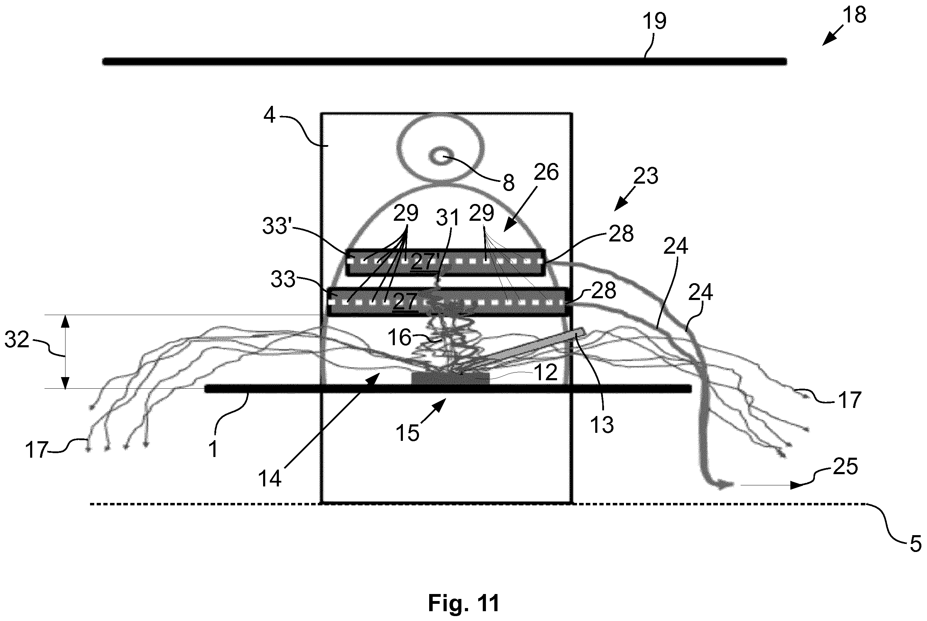

Diese Wirksamkeit kann durch weitere Modifikationen an der angegebenen Vorrichtung 23 weiter gesteigert werden. Diese Modifikationen sollen anhand der Figuren 11 bis 14 näher erläutert werden.

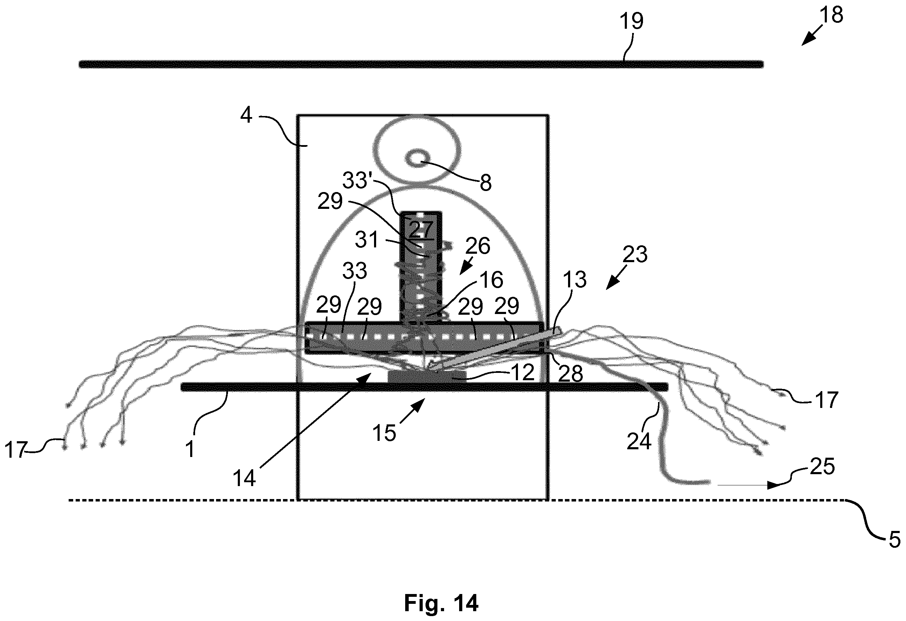

Eine Unterscheidung zwischen dem Begriff Kammer 27 und schlauchförmiger Körper 33 ist zunächst in den Fig. 11 bis 13 eher unerheblich. Ihr Sinn wird erst später bei der Beschreibung in Fig. 14 klar.

Wie in Fig. 11 gezeigt, kann von der Emissionsquelle 15 aus gesehen über der bereits in Fig. 8 und Fig. 9 erläuterten Kammern 27 eine weitere Kammer 27' angeordnet werden, die den die Kammer 27 passierenden Rest 31 an chirurgischem Rauch 14 über ihre Ansaugöffnungen 29 ansaugt. Sollte die weitere Kammer 27' immer noch nicht ausreichend sein, die Gesamtbelastung an über die Inhalationsöffnung 8 inhalierten Partikeln des chirurgischen Rauches 14 unterhalb einer vorbestimmten Grenze zu halten, dann können über der weiteren Kammer 27' selbstverständlich weitere Kammern 27 angeordnet werden.

Die einzelnen Kammern 27, 27' können, wie in Fig. 11 gezeigt über ihre Absaugschläuche 24 an eine gemeinsame Unterdruckquelle 25 oder an jeweils individuelle Unterdruckquellen 25 angeschlossen werden. Dabei können die einzelnen Absaugschläuche 24 auch auf halben Weg beispielsweise Y- oder anderweitig baumförmig ineinander laufen.

Sind die Kammern 27, 27' in der bereits erwähnten Weise als schlauchförmige Körper 33, 33' ausgeführt, dann können die einzelnen schlauchförmigen Körper 33, 33' in einer beliebigen relativen Lage zueinander angeordnet werden. In Fig. 11 sind die gezeigten schlauchförmigen Körper 33, 33' parallel zueinander angeordnet.

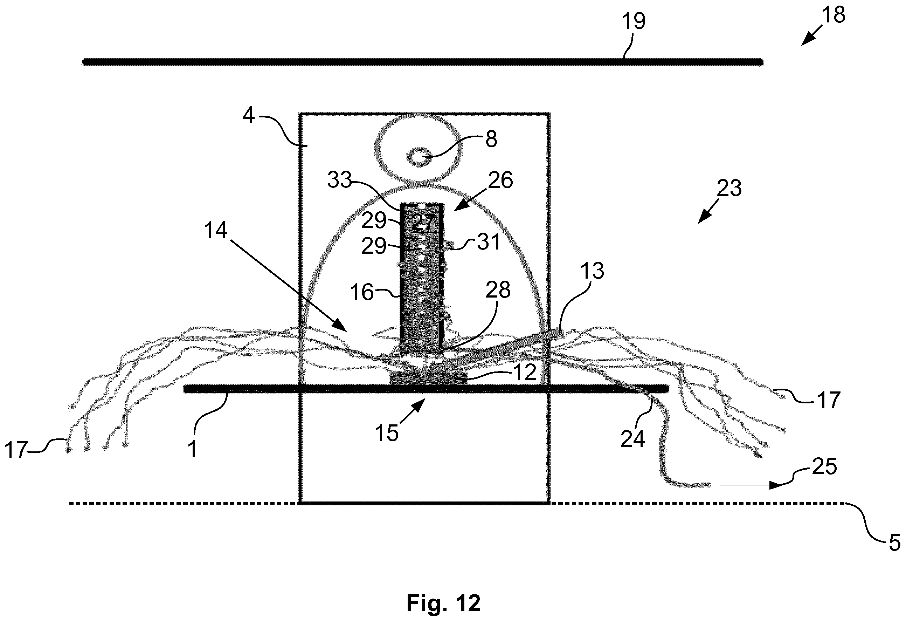

Unabhängig davon kann jedoch auch die relative Lage einer einzelnen Kammer 27 und insbesondere eines schlauchförmigen Körpers 33 gegenüber der Emissionsquelle 15 beliebig sein. Während der schlauchförmige Körper 33 beispielsweise Fig. 11 horizontal über dem Boden 5 verläuft, kann der schlauchförmige Körper 33 wie in Fig. 12 gezeigt, auch vertikal über dem Boden 5 verlaufen.

Auf diese Weise wird zwar die Wirkungsfläche der angegebenen Vorrichtung 23 vom Boden 5 aus gesehen über der Emissionsquelle 15 reduziert, jedoch wird der heiße chirurgische Rauch 16 über eine längere Strömungsstrecke wirksamer abgesaugt.

Wie in Fig. 13 gezeigt, kann bei einer vertikalen Anordnung des schlauchförmigen Körpers 33 ein weiterer schlauchförmiger Körper 33' parallel daneben verlaufend angeordnet werden.

Eine einzelne Kammer 27 kann aber, wie in Fig. 14 gezeigt, auch aus mehreren, insbesondere winklig zueinander angeordneten schlauchförmigen Körpern 33, 33' gebildet sein. Diese schlauchförmigen Körper 33, 33' sind dann ineinander miteinander verbunden, so dass gegebenenfalls nur ein einziger Absaugschlauch 24 notwendig ist, um den Unterdruck der Unterdruckquelle 25 an die angegebene Vorrichtung 23 vollständig anzulegen.

Eine winklige Anordnung der beiden schlauchförmigen Körper relativ zueinander hat zudem den Vorteil, dass die große Wirkfläche gemäß Fig. 11 mit der langen Wirkung über die Strömungsstrecke des heißen chirurgischen Rauches 16 miteinander kombiniert werden kann.

Nachstehend soll anhand von Fig. 15 eine als schlauchförmiger Körper 33 ausgeführte Kammer 27 und ihre Befestigung am Operateur 4 anhand einer Schnittdarstellung näher erläutert werden.

Wie bereits erläutert, kann die Kammer 27, die nachstehend in nicht einschränkender Weise als aus einem einzigen schlauchförmigen Körper 33 ausgebildet angenommen werden soll, über das Befestigungselement 30 am Operateur 4 befestigt sein.

Das Befestigungselement 30 kann dabei grundsätzlich beliebig aufgebaut sein. Applikationsabhängig hat sich in den durchgeführten Experimenten gezeigt, dass ein selbstklebendes Befestigungselement 30 in Form eines Kunststoffstreifens mit einer selbstklebenden Oberfläche am sinnvollsten ist. Je nachdem, wie und wo die angegebene Vorrichtung 23 am Operateur 4 befestigt werden soll, kann das Befestigungselement 30 jedoch auch andere Befestigungsmechanismen realisieren.

Das Befestigungselement 30 befestigt den schlauchförmigen Körper 33 in einer Befestigungsebene 35. Gegenüber dieser Befestigungsebene 35 können die einzelnen Ansaugöffnungen 29, wie bereits erwähnt, mit einem beliebigen Ansaugwinkel 34 in die durch den schlauchförmigen Körper 33 gebildeten Kammer 27 eintreten, was zu unterschiedlichen Einströmungsrichtungen für den chirurgischen Rauch 14 in die Kammer 27 führt. Diese Einströmrichtungen sind in Fig. 15 mit einem Pfeil dargestellt und der Übersichtlichkeit halber mit dem Bezugszeichen für den chirurgischen Rauch 14 versehen.

Die im Rahmen der Fig. 9 und 10 durchgeführten Versuche wurden zusätzlich noch einmal mit verschiedenen Ansaugwinkeln 34 durchgeführt. Dabei wurden als Abstand 32 wieder 15 cm (entspricht 0,15 m) und als Volumenstrom 22 330 l/min (entspricht 19,3 m³/h) gewählt.

Mit einem Ansaugwinkel 34 von 45° wurde dabei die bereits in Fig. 10 gezeigte Absaugwirkung 21 von 99,2% erreicht, während mit einem Ansaugwinkel 34 von 0° (d.h. die Einströmrichtung des chirurgischen Rauches 14 verläuft in der Befestigungsebene 35) eine Absaugwirkung 21 von nur 97 % erreicht wurde. Noch weiter fiel die Absaugwirkung 21, bei einem Ansaugwinkel von 90° (d.h. die Einströmrichtung des chirurgischen Rauches 14 verläuft rechtwinklig zur Befestigungsebene 35) auf einen Wert von 86 %.

In der Regel trägt der Operateur 4 ein Kleidungsstück 36, wie beispielsweise eine sterile Schutzschürze. Im Rahmen der obigen Versuche konnte nachgewiesen werden, dass die Oberflächenstruktur des Kleidungsstücks 36 ebenfalls einen Einfluss auf die Absaugwirkung 21 hat. Wies das Material des Kleidungsstücks 36 eine raue Oberfläche, wie beispielsweise bei Frottee oder Baumwolle-Polyester-Mischungen auf, so war die Absaugwirkung 21 um bis zu 43% niedriger als bei einer glatten Oberfläche, an Propylenfolien oder glattem Papier.

Daher wird im Rahmen der vorliegenden Ausführung ein optionales zusätzliches Trägerelement 37 vorgeschlagen, das auf das Kleidungsstück 36 aufsetzbar ist. Es kann eine gegebenenfalls vorhandene rauhe Oberfläche des Kleidungsstückes 36 glätten und so die Absaugwirkung 21 weiter steigern. Als Material für dieses Trägerelement 37 eignet sich insbesondere eine Kunststofffolie.

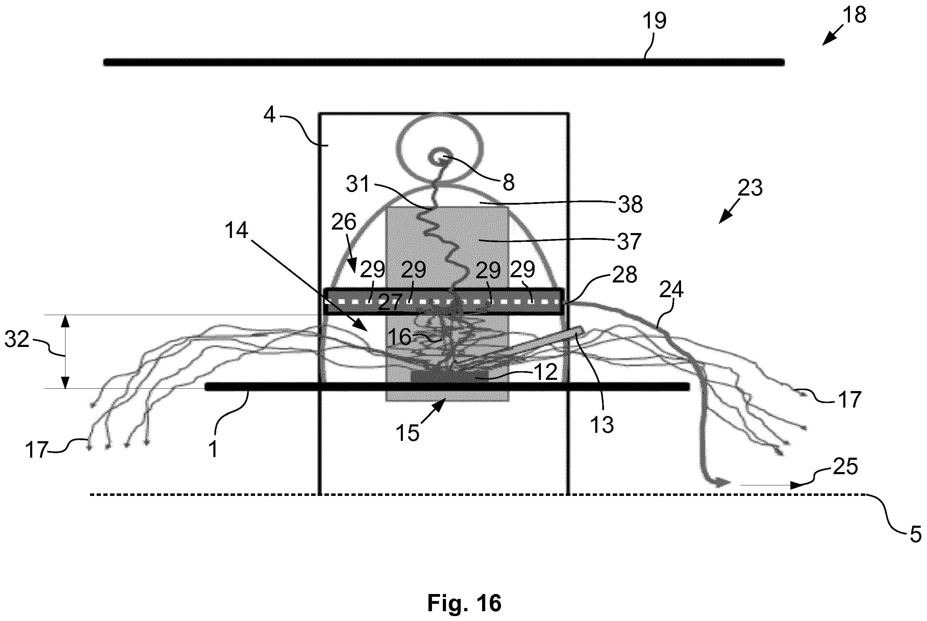

Das Trägerelement 37 sollte sich möglichst vollständig über den Strömungsweg des chirurgischen Rauchs 14 zwischen der Emissionsquelle 15 und der Inhalationsöffnung 8 erstrecken. Deshalb sollte das Trägerelement 37 so ausgeführt sein, dass es sich wenigstens von der Emissionsquelle 15 bis zu einem in Fig. 16 angedeuteten Schulterbereich 38 des Operateurs 4 erstreckt.

Zur weiteren Verbesserung der Absaugwirkung 21 der angegebenen Vorrichtung 23 kann gesehen von der Emissionsquelle 15 aus der schlauchförmige Körper 33 mit einem Abdeckelement 39 abgedeckt werden. Dieses Abdeckelement stellt für den strömenden chirurgischen Rauch 14 im Bereich der Kammer 27 ein Strömungshindernis dar und führt zu einer turbulenten Strömung. Auf diese Weise wird der strömende chirurgische Rauch 14 im Bereich der Kammer 27 verlangsamt, wodurch er effektiver in die Ansaugöffnungen 29 gelangen kann.

Auf einen möglichen Aufbau des Abdeckelements 39 wird an späterer Stelle noch im Detail näher eingegangen.

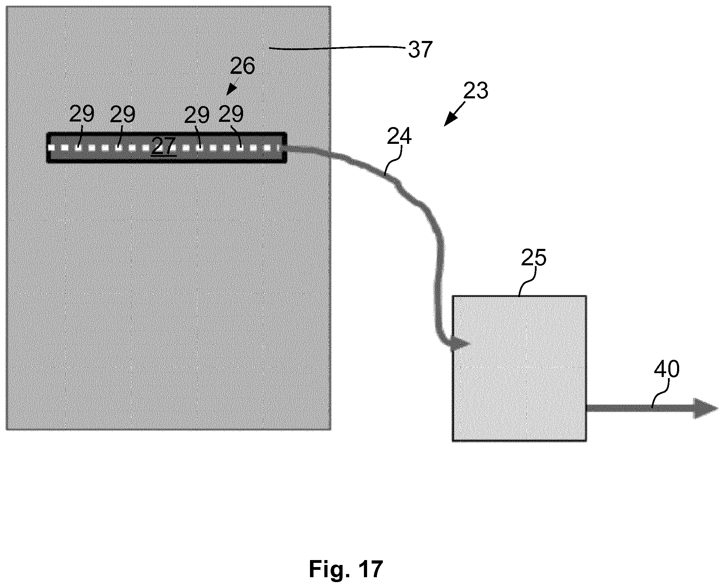

Die gesamte angegebene Vorrichtung 23 ist auf dem Trägerelement 37 getragen in Fig. 17 dargestellt. Die Unterdruckquelle 25 kann dabei beliebig aufgebaut sein. Sie kann einen nicht weiter dargestellten Filter zum Abscheiden des chirurgischen Rauchs umfassen. Weiterhin kann die Unterdruckquelle 25 eine nicht weiter dargestellte Pumpe zur Erzeugung des Unterdruckes umfassen. Die zur Erzeugung des Unterdruckes abgesaugte Luft kann schließlich über einen Abluftkanal 40 ausgestoßen werden.

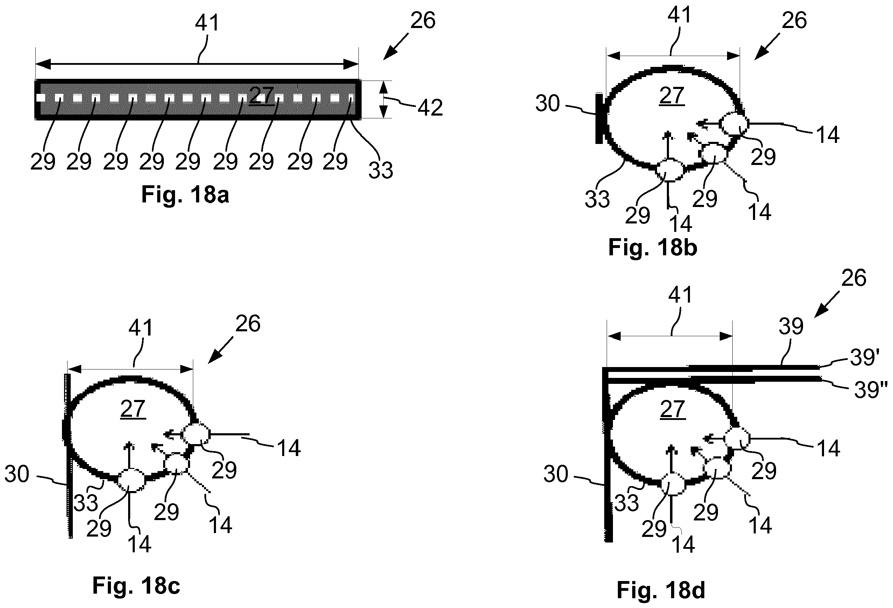

Nachstehend sollen verschiedene Ausführungen des Absaugelementes 26 der angegebenen Vorrichtung 23 anhand der Fig. 18A bis 20K näher erläutert werden.

In Fig. 18A ist das Absaugelement 26 in der Konfiguration gezeigt, wie es für die obigen Experimente verwendet wurde, d.h. die Kammer 27 des Absaugelements 26 ist durch einen schlauchförmigen Körper 33 mit einem kreisrunden Querschnitt gebildet. Der schlauchförmige Körper 33 weist eine Körperlänge 41 von 400 mm und einen Körperdurchmesser 42 von 22 mm auf, was einer Rohrfläche von 380 mm² entspricht. Auf der Mantelfläche des schlauchförmigen Körpers 33 ist axial verlaufend eine Reihe von 95 äquidistant zueinander angeordneten Ansaugöffnungen 29 mit einem Abstand von jeweils 4 mm zueinander angeordnet. Die einzelnen Ansaugöffnungen 29 sind mit einem viereckigen Querschnitt dargestellt. Für die obigen Experimente wurde jedoch für die Ansaugöffnungen 29 ein kreisrunder Querschnitt mit je einem Durchmesser von 2 mm verwendet. Auf diese Weise ergab sich eine Gesamtfläche aller Ansaugöffnungen von ca. 295 mm².

Wie bereits im Rahmen der Fig. 15 erläutert, können die Ansaugöffnungen 29 mit einem beliebigen Ansaugwinkel 34 zur Befestigungsebene 35 angeordnet werden. Es ist ebenfalls möglich, die Ansaugöffnungen 29 mehrreihig in verschiedenen Ansaugwinkeln 34 anzuordnen. Ferner ist es möglich, die Ansaugöffnungen 34 über verschiedene Ansaugwinkel 34 verteilt anzuordnen.

Das Befestigungselement 30 kann, wie in in den Fig. 18B und 18C gezeigt, beliebig groß ausgebildet sein. Je größer das Befestigungselement 30 ist, desto sicherer ist der Halt des schlauchförmigen Körpers 33 am Operateur 4.

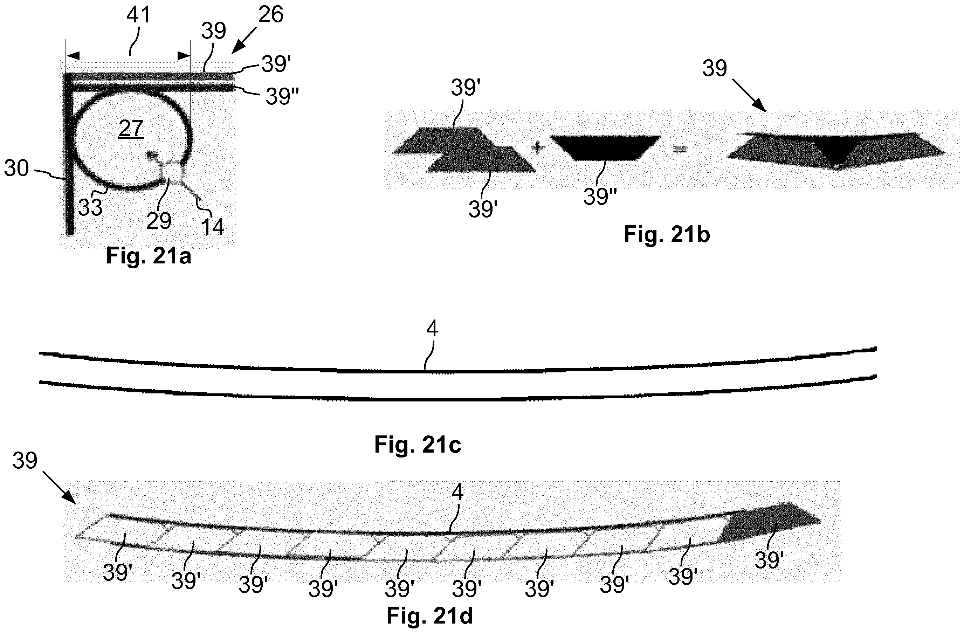

Das Abdeckelement 39 kann mehrteilig aufgebaut sein, worauf in Fig. 21A bis 22C an späterer Stelle näher eingegangen wird. Die einzelnen Abdeckelementteile 39', 39" des mehrteiligen Abdeckelementes 39 können, wie in Fig. 18D gezeigt, in verschiedenen Ebenen angeordnet sein.

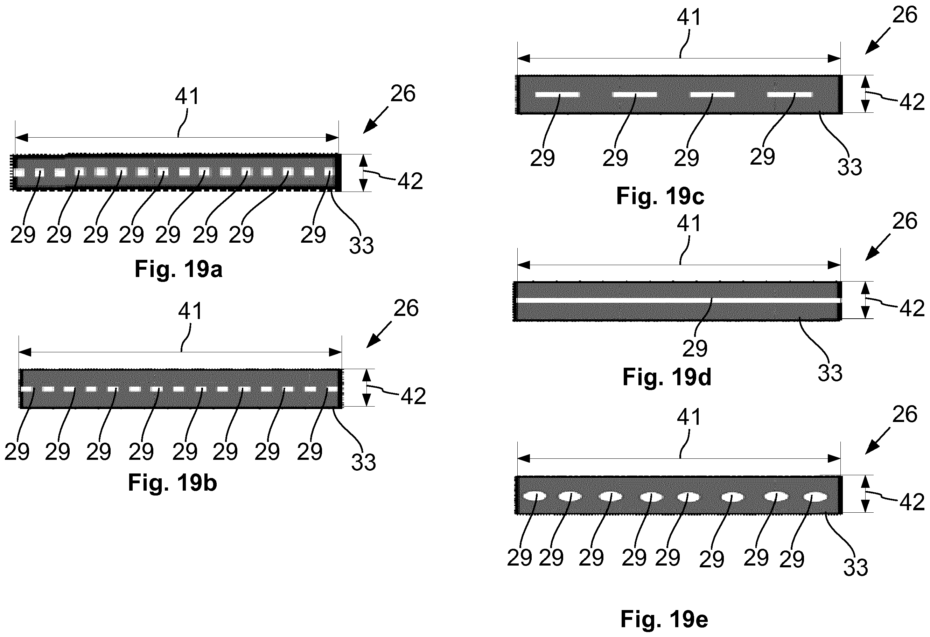

Es wird auf die Fig. 19A bis 19E Bezug genommen, in denen verschiedene Ausführungen für die Ansaugöffnung 29 des Absaugelementes 26 angedeutet sind. Gegenüber der bereits erläuterten Ausführung gemäß Fig. 19A können die Ansaugöffnungen 29 auch in Umfangsrichtung des schlauchförmigen Körpers 33 (wie in Fig. 19B gezeigt) oder in Axialrichtung des schlauchförmigen Körpers 33 (wie in Fig. 19C gezeigt) schlitzförmig verlaufend ausgebildet sein. Dabei kann auch als Ansaugöffnung 29 ein einziger durchlaufender Schlitz, wie in Fig. 19D gezeigt, angeordnet werden. Ferner ist es ebenfalls möglich, wie in Fig. 19E gezeigt, einen elliptischen Querschnitt für die Ansaugöffnungen 29 zu wählen.

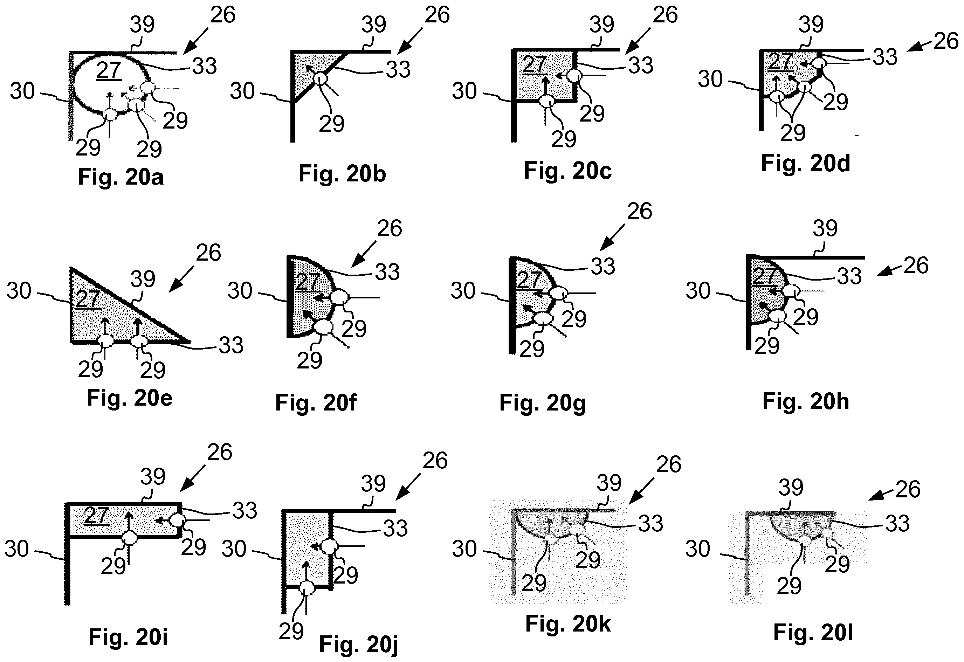

In den Fig. 20A bis 20L sind verschiedene Querschnitte und Ausführungen für den schlauchförmigen Körper 33 gezeigt. In der in Fig. 20A gezeigten, bereits diskutierten Ausführung ist der schlauchförmige Körper 33 einstückig mit einem kreisrunden Querschnitt aufgebaut. Alternativ kann der schlauchförmige Körper 33 aber auch mehrstückig aufgebaut werden, wobei das Abdeckelement 39 und/oder das Befestigungselement 30 den schlauchförmigen Körper 33 begrenzen können. Dabei können verschiedene in den Fig. 20B bis 20L gezeigte Querschnitte geformt werden. Gegebenenfalls können auch das Abdeckelement 39 und das Befestigungselement 30 einstückig ausgeführt werden.

Nachstehend wird anhand der Fig. 21A bis 22C ein Ausführungsbeispiel für das Abdeckelement 39 näher beschrieben.

Hierbei können die einzelnen Abdeckteilelemente 39',39" wie in Fig 21b gezeigt, trapezförmig ausgeführt werden. Dabei können die Abdeckteilelemente 39',39" in den einzelnen Ebenen jeweils gleichlagig aber zueinander verschoben angeordnet sein, wobei die Abdeckteilelemente 39', 39" zwischen den Ebenen um 180° gedreht sein können. Auf diese Weise wird erreicht, dass sich das resultierende Abdeckelement 39, wie in den Fig. 21C und 21D gezeigt, optimal der Körperform des Operateurs 4 anpasst.

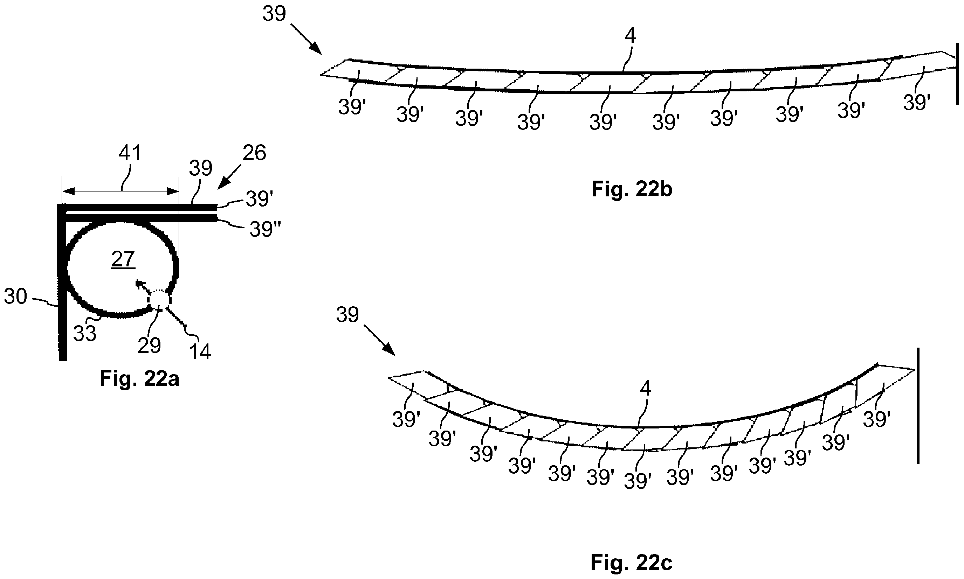

Wie insbesondere in den Fig. 22B und 22C verdeutlicht, ist ein derartiges, mehrteiliges Abdeckelement 39 extrem flexibel und passt sich selbst an stark veränderliche Körperformen des Operateurs 4 an.

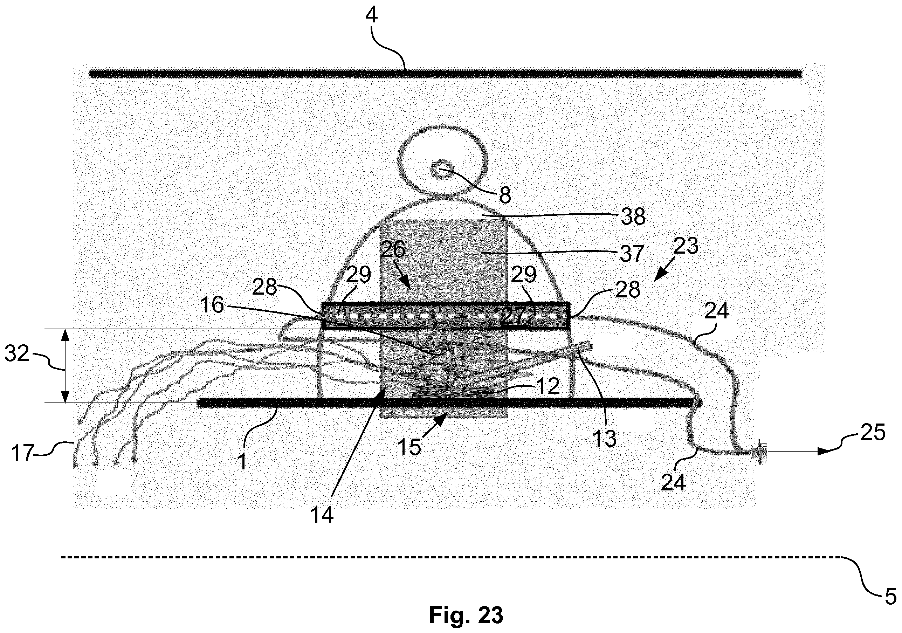

Abschließend sei noch auf die Alternative hingewiesen, dass an eine einzelne Kammer 27 auch mehrere Absaugschläuche 24 angeschlossen werden können, wie in Fig. 23 gezeigt.

## Patentansprüche

1. Vorrichtung (23) zur Absaugung von chirurgischem Rauch (14) genannten partikulären und/oder gasförmigen Emissionen zwischen einer Emissionsquelle (15) im Frontbereich eines Operateurs (4) und dessen Mund-Nasen-Bereich (8), umfassend
- einen Absaugschlauch (24) zur Bereitstellung eines Unterdruckes aus einer Unterdruckquelle (25), und
- ein Absaugelement (26) mit
- einer durch wenigstens einen schlauchförmigen Körper (33) gebildeten Kammer (27) mit einer Schlauchquerschnittsfläche,
- einer die Kammer (27) öffnenden Absaugöffnung (28), an die der Absaugschlauch (24) zum Erzeugen des Unter druckes in der Kammer (27) anschließbar ist, und
- einer Vielzahl die Kammer (27) öffnenden und den Mantel des schlauchförmigen Körpers (33) durchdringenden Ansaugöffnungen (29) zum Ansaugen des chirurgischen Rauchs (14) basierend auf dem Unterdruck in der Kammer (27),
**dadurch gekennzeichnet, dass** jede Ansaugöffnung (29) eine Öffnungsquerschnittsflächen aufweist, und wobei die Summe aller Öffnungsquerschnittsflächen kleiner ist, als die 2,5-fache Schlauchquerschnittsfläche.

2. Vorrichtung (23) nach Anspruch 1, wobei die Summe aller Öffnungsquerschnittsflächen aus einem Bereich zwischen dem 0,5-fachen und dem 2,5-fachen der Schlauchquerschnittsfläche ausgewählt ist.

3. Vorrichtung (23) nach Anspruch 1 oder 2, wobei jede Querschnittfläche kleiner ist als 10% der Summe aller Öffnungsquerschnittsflächen.

4. Vorrichtung (23) nach einem der vorstehenden Ansprüche, wobei die Kammer (27) durch wenigstens einen schlauchförmigen Körper (33) gebildet ist, dessen Mantel von den Ansaugöffnungen (29) durchdrungen ist.

5. Vorrichtung (23) nach Anspruch 4, wobei die Kammer (27) durch wenigstens einen weiteren schlauchförmigen Körper (33') gebildet ist, der vorzugsweise winklig zum ersten schlauchförmigen Körper (33) angeordnet ist.

6. Vorrichtung (23) nach Anspruch 4 oder 5, wobei der schlauchförmige Körper (33) im Profil rund und/oder eckig gebildet ist.

7. Vorrichtung (23) nach einem der vorstehenden Ansprüche, wobei das Verhältnis zwischen den Mindestabständen der Ansaugöffnungen (29) in Millimetern und den Querschnittsflächen der Ansaugöffnungen (29) in Quadratmillimetern untereinander zwischen 1/50 und 1/200, vorzugsweise 1/100 beträgt.

8. Vorrichtung (23) nach einem der vorstehenden Ansprüche, umfassend ein Befestigungselement (30) zur Befestigung des Absaugelementes (26) am Operateur (4).

9. Vorrichtung (23) nach Anspruch 8, wobei die Ansaugöffnungen (29) zu einer Befestigungsebene (35), in der das Befestigungselement (30) eingerichtet ist, das Absaugelement (26) am Operateur (4) zu befestigen, mit einem Winkel (34) zwischen -90° und 90°, insbesondere 45°, verlaufen.

10. Vorrichtung (23) nach Anspruch 8 oder 9, wobei das Befestigungselement (30) eingerichtet ist, das Absaugelement (26) selbstklebend am Operateur (4) zu befestigen.

11. Vorrichtung (23) nach einem der vorstehenden Ansprüche 8 bis 10, wobei das Befestigungselement (30) einen Folienkörper umfasst.

12. Vorrichtung (23) nach einem der vorstehenden Ansprüche, wobei ein Material des Absaugelements (26) Kunststoff und/oder Edelstahl umfasst.

13. Arbeitskleidung für einen Operateur (4), umfassend ein Kleidungsstück (36) zur wenigstens teilweisen Bedeckung des Operateurs (4) und eine Vorrichtung (23) nach einem der vorstehenden Ansprüche, die über ihr Befestigungselement (30) an einer vom Operateur (4) aus gesehenen Außenseite des Kleidungsstückes (36) an diesem befestigt ist.

14. Arbeitskleidung nach Anspruch 13, umfassend ein zwischen dem Befestigungselement (30) und dem Kleidungsstück (36) angeordnetes Trägerelement (37).

15. Arbeitskleidung nach Anspruch 14, wobei das Trägerelement (37) selbstklebend und/oder ein Zuggurt ist.

16. Arbeitskleidung nach Anspruch 14 oder 15, wobei sich das Trägerelement (37) von einem Schulterbereich (38) des Operateurs (4) bis unterhalb der Emissionsquelle (15) erstreckt.

## Claims

1. Device (23) for suctioning of particulate and/or gaseous emissions named surgical smoke(14) between an emission source (15) in the front area of a surgeon (4) and its mouth-nose-area (8), comprising:
- an suctioning hose (24) for providing a negative pressure from a negative pressure source (25), and
- a suctioning element (26) with
- a chamber (27) formed by at least one hose like body (33) and having a hose cross section area,
- suction openings (28) opening the chamber (27), wherein the suctioning hose (24) is connectable to the suctioning openings (28) for generating the negative pressure in the chamber (27), and
- a plurality of aspiration openings (29) that opens the chamber (27) and passes through the boundary of the hose like body (33) for aspirating the surgical smoke (14) based on the negative pressure in the chamber (27),
**characterized in that**
each aspiration opening (29) has an opening sectional area, and wherein the sum of all opening sectional area is smaller than 2,5 times of the hose cross section area.

2. Device (23) according to claim 1, wherein the sum of all opening sectional areas is chosen from an interval between 0,5 times and 2,5 times of the hose cross section area.

3. Device (23) according to claim 1 or 2, wherein each opening sectional area is less than 10% of the sum of all opening sectional areas.

4. Device (23) according to one of the preceding claims, wherein the chamber (27) is formed by at least one hose like body (33), which boundary is passed through by the aspiration openings (29).

5. Device (23) according to claim 4, wherein the chamber (27) is formed by at least one further hose like body (33') that is preferably arranged to the first hose like body (33) at an angle.

6. Device (23) according to claim 4 or 5, wherein the hose like body (33) in formed round an/or angularly in profile.

7. Device (23) according to one of the preceding claims, wherein the ratio between the minimum distances of the aspiration openings (29) in millimeters and the sectional areas of the aspiration openings (29) in square millimeters amongst each other is chosen between 1/50 and 1/200, preferably by 1/100.

8. Device (23) according to one of the preceding claims, comprising a mounting element (30) for mounting the suctioning element (26) at the surgeon (4).

9. Device (23) according to claim 8, wherein the aspiration openings (29) run to a mounting plane (35), in which the mounting element (30) adapted to mount the suctioning element (26) at the surgeon (4), in an angle (34) between -90° and 90°, in particular with 45°.

10. Device (23) according to claim 8 or 9, wherein the mounting element (30) is adapted to mount the suctioning element (26) at the surgeon (4) in a self-adhesive way.

11. Device (23) according to one of the claims 8 to 10, wherein the mounting element (30) comprises a foil body.

12. Device (23) according to one of the preceding claims, wherein the material of the suctioning element (26) is plastic and/or stainless steel.

13. Work wear for a surgeon (4), comprising a wear piece (36) for covering the surgeon (4) at least partly and a device (23) according to one of the preceding claims that is fixed at the wear piece (36) via its mounting element (30) at its outside seen from the surgeon (4).

14. Work war according to claim 13, comprising a supporting element (37) that is arranged between the mounting element (30) and the wear piece (36).

15. Work wear according to claim 14, wherein the supporting element (37) is self-adhesive and/or a tension flange.

16. Work wear according to claim 14 or 15, wherein the supporting element (37) extents from a shoulder area (38) of the surgeon (4) up to underneath the emission source (15).

## Revendications

1. Dispositif (23) pour extraire la fumée chirurgicale (14) ou émission de particules et/ou de gaz entre une source d'émission (15) dans la région frontale d'un chirurgien (4) et la région de sa bouche et de son nez (8), comprenant :
- un tuyau d'aspiration (24) pour créer une pression négative à partir d'une source de vide (25), et
- un élément d'aspiration (26) comportant
- une chambre (27) formée par au moins un corps tubulaire (33) ayant une surface de section transversale de tube,
- une ouverture d'aspiration (28) qui s'ouvre sur la chambre (27) et à laquelle peut être connecté le tuyau d'aspiration (24) pour générer la pression négative dans la chambre (27), et
- une pluralité d'orifices d'admission (29) s'ouvrant dans la chambre (27) et traversant la paroi du corps tubulaire (33) pour aspirer la fumée chirurgicale (14) par l'effet de la pression négative dans la chambre (27),
**caractérisé en ce que**
chaque orifice d'admission (29) a une surface de section transversale d'ouverture, et dans lequel la somme de toutes les surfaces de section transversale d'ouverture est plus petite que 2.5 fois la surface de section transversale de tube.

2. Dispositif (23) selon la revendication 1, dans lequel la somme de toutes les surfaces de section transversale d'ouverture est choisie dans une plage comprise entre 0.5 fois et 2.5 fois la surface de section transversale de tube.

3. Dispositif (23) selon la revendication 1 ou la revendication 2, dans lequel chaque surface de section transversale est plus petite que 10 % de la somme de toutes les surfaces de section transversale d'ouverture.

4. Dispositif (23) selon l'une quelconque des revendications précédentes, dans lequel la chambre (27) est formée d'au moins un corps tubulaire (33), dont la paroi est traversée par les orifices d'admission (29).

5. Dispositif (23) selon la revendication 4, dans lequel la chambre (27) est formée par au moins un corps tubulaire supplémentaire (33'), lequel est de préférence orienté angulairement par rapport au premier corps tubulaire (33).

6. Dispositif (23) selon la revendication 4 ou la revendication 5, dans lequel le corps tubulaire (33) est formé selon un profil arrondi et/ou carré.

7. Dispositif (23) selon l'une quelconque des revendications précédentes, dans lequel le rapport entre les distances minimales des orifices d'admission (29) en millimètres et la surface de section transversale des orifices d'admission (29) en millimètres carrés est compris entre 1/50 et 1/200, de préférence de 1/100.

8. Dispositif (23) selon l'une quelconque des revendications précédentes, comprenant un élément de fixation (30) pour fixer l'élément d'aspiration (26) sur le chirurgien (4).

9. Dispositif (23) selon la revendication 8, dans lequel les orifices d'admission (29) sont solidarisés à un plan de fixation (35), dans lequel l'élément de fixation (30) est structuré pour fixer l'élément d'aspiration (26) au chirurgien (4) selon un angle (34) compris entre -90° et 90°, en particulier de 45°.

10. Dispositif (23) selon la revendication 8 ou la revendication 9, dans lequel l'élément de fixation (30) est adapté pour solidariser l'élément d'aspiration (26) au chirurgien (4) de façon auto adhésive.

11. Dispositif (23) selon l'une quelconque des revendications précédentes 8 à 10, dans lequel l'élément de fixation (30) comprend un corps en forme de film.

12. Dispositif (23) selon l'une quelconque des revendications précédentes, dans lequel un matériau de l'élément d'aspiration (26) comprend la matière plastique et/ou l'acier inoxydable.

13. Vêtement de travail pour un chirurgien (4), comprenant une pièce de vêtement (36) pour une couverture au moins partielle du chirurgien (4) et un dispositif (23) selon l'une quelconque des revendications précédentes, lequel dispositif est solidarisé à l'opérateur par son élément de fixation (30) sur l'un des côtés de la pièce de vêtement (36) visibles par le chirurgien (4).

14. Vêtement de travail selon la revendication 13, comprenant un élément support (37) disposé entre l'élément de fixation (30) et la pièce de vêtement (36).

15. Vêtement de travail selon la revendication 14, dans lequel l'élément support (37) est un autocollant et/ou une sangle de serrage.

16. Vêtement de travail selon la revendication 14 ou la revendication 15, dans lequel l'élément support (37) s'étend depuis une zone d'épaule (38) du chirurgien (4) jusqu'à au-dessous de la source d'émission (15).
